# EUROPEAN PATENT APPLICATION

(11) **EP 4 678 115 A1**
(43) Date of publication of application: **14.01.2026**
(21) Application number: 24306149.6
(22) Date of filing: 09.07.2024
(51) Int. Cl.: A61B 8/08, A61B 5/00

(54) **ELASTOGRAPHY DEVICE AND METHOD**

(71) Applicant: Echosens, 75014 Paris (FR)
(72) Inventor: Sandrin, Laurent, 92340 Bourg-la-Reine (FR); Audière, Stéphane, 75012 Paris (FR); Miette, Véronique, 94240 L'Hay-les-Roses (FR); Lacoin, Guillaume, 75014 Paris (FR); Bastard, Cécile, 75012 Paris (FR)
(74) Representative: Cabinet Camus Lebkiri

(57) **Abstract**

The present invention relates an elastography method comprising:
- acquire, at a repetition rate of at least 4 measurements per second and for a duration of at least 3 seconds, measurements of a mechanical property of a region of a body of a subject, said region being a part of a liver or a part of a spleen of the subject;
- obtain a signal representative of the variations of the mechanical property with time, said signal comprising at least part of the measurements of the mechanical property determined;
- in each portion of the signal among at least one portion of the signal, select measurements, among the measurements comprised in the portion of the signal, lower than or equal to a threshold associated with the portion of the signal; and
- determine a value representative of the mechanical property, called resting value, based on the selected measurements.

## Description

### FIELD

The present invention relates to the field of elastography, for example liver or spleen elastography. More specifically, the invention relates to an elastography device and an elastography method to accurately determine a measurement of the liver stiffness or the spleen stiffness of a subject.

### BACKGROUND

Measuring liver stiffness (LS) has been shown to be a very useful tool to help health care professionals detect or characterize liver disease or damages, and more generally monitor the condition of the liver of a subject.

There are basically two kinds of elastography techniques for measuring stiffness: strain imaging and shear wave imaging. In strain imaging, a normal stress is applied to the tissue and the normal strain is measured. The Young's modulus is then determined from the applied normal stress and the measured normal strain. In shear wave imaging, a dynamic stress is applied to the tissue and shear waves created by the excitation are then followed to derive a measurement of the stiffness of the tissue. Techniques based on shear wave imaging include Point Shear Wave Elastography (pSWE), 2D Shear Wave Elastography (SWE) and 1D Transient Elastography (TE). An example of 1D Transient Elastography is the Vibration-Controlled Transient Elastography (VCTE) used in the FibroScan^{®} system.

The VCTE-based elastography devices typically provide several measurements of the stiffness of the tissue (for example the liver or the spleen) collected at different timepoints when the operator triggers measurements (for example by pressing a button to generate a transient displacement). A unique value representative of the stiffness measurement is then derived from these measurements (for example, the final measurement may be a mean or a median of the series of measurements). To obtain a reliable measurement of liver stiffness, it is generally recommended to obtain a series of 10 stiffness measurements.

Even if this technology works well and provides very satisfying results, it is known that variability in the series of stiffness measurements can be quite large.

There are many factors that can influence the stiffness value of certain organs, such as the liver or the spleen, and which can therefore induce variability in the stiffness measurements. For example, liver stiffness measurements (LSM) or spleen stiffness measurements (SSM) are influenced by central venous pressure and more generally by hemodynamics effects, as detailed in the article "Liver Stiffness Is Directly Influenced by Central Venous Pressure" by G. Millonig et al., Journal of Hepatology, volume 52, Issue 2, February 2010, pp. 206-210.

The inventors of the present invention investigated whether other factors could explain this variability, and how such factors might be considered in the evaluation of the liver stiffness or spleen stiffness of the subject, for example to assess liver fibrosis or portal hypertension, respectively.

### SUMMARY

An aspect of the invention relates to an elastography method and an elastography device for determining a value representative of a mechanical property of a region of a liver or a spleen of a subject, called "resting value", which is not influenced by the respiration of the subject, pressure effects or the inclination of the probe. The inventors of the present invention have in fact determined that all these factors can lead to overestimations of the measurement of the mechanical property. According to the invention, the resting value is therefore determined by removing overestimations associated with the inclination of the probe (it has in fact been observed that when the probe is not perpendicular to the subject's body, the measurement acquired is higher than when the probe is perpendicular to the subject's body), the subject's respiration and the blood pressures. The resting value is in fact much more correlated with the level of fibrosis of the subject than the current measurement techniques. The diagnostic value of the value thus obtained is therefore greatly increased. In addition, the elastography method according to the invention allows a novice operator to perform an examination with similar quality that an experienced operator. Another advantage is that the elastography method of the invention makes it possible to overcome measurement variations linked to the subject's breathing, without the subject having to hold their breath (which can be complicated, particularly for overweight subjects).

An aspect of the invention therefore relates to an elastography method implemented by computer, comprising:
- acquiring, at a repetition rate of at least 4 measurements per second and for a duration of at least 3 seconds, measurements of a mechanical property of a region of a body of a subject, said region being a part of a liver or a part of a spleen of the subject;
- obtaining a signal representative of the variations of the mechanical property with time, said signal comprising at least part of the measurements of the mechanical property determined;
- in each portion of the signal among at least one portion of the signal, selecting measurements, among the measurements comprised in the portion of the signal, lower than or equal to a threshold associated with the portion of the signal; and
- determining a value representative of the mechanical property, called resting value, based on the selected measurements.

By "mechanical property", it is meant any physical property or parameter relating to the behavior of the region of the human body when subjected to one or more mechanical stresses. For example, the mechanical property may be conventional quantities such as stiffness, elasticity, Young's modulus, shear modulus, shear wave speed, viscoelasticity, viscosity, etc. The mechanical property may also be any value derived from one or more of these physical properties or parameters.

In the following, particular reference is made to stiffness, but aspects of the invention apply to other mechanical properties. Also, particular reference is made to the liver as a region of the body, but aspects of the invention could apply to the spleen.

By "repetition rate", it is meant a number of measurements performed by unit of time. By "duration" it is meant a time interval during which the measurements are acquired.

In the present invention, this acquisition rate is at least 4 measurements per second, to acquire measurements with a sufficiently high repetition rate to be able to observe the variations of the signal during a respiratory cycle. For this reason, the measurements are acquired during at least 3 seconds, which corresponds to the average respiratory cycle of an adult. It will be appreciated the duration of the acquisition may be much longer than 3 seconds. In particular, the duration of the acquisition may be at least 20 seconds, to observe variations in the measurements of the mechanical property over several respiratory cycles.

To increase the precision of the constructed signal, the measurements may be acquired at shorter times, i.e. at a higher repetition rate, for example at least 5 measurements per second (e.g. 5, 6, 7, 8, 9, 10 measurements per second) or more than 10 measurements per second. The higher the repetition rate, the more accurately the variations of the signal are captured, but the greater the computational resources are to be implemented.

According to the elastography method introduced above, in each portion of the signal among the at least one portion of the signal, measurements greater than the threshold associated with the portion of the signal are suppressed. The "high" values (overestimations) of the signal are therefore removed from the signal to determine the resting value.

The inventors have indeed devised that the signal included a "low" component, which corresponds to a component which is not linked to the subject's breathing, pressure effects or probe perpendicularity. Therefore, to extract this component from the signal, only "low" values of the signal are selected by applying a threshold, and the resting component is constructed from these low values alone (and not from other values).

By "at least one portion of the signal", it is meant one portion of the signal (for example, the signal itself) or several portions of the signal. In the case of a plurality of portions of the signal, these portions of the signal may partially overlap, or not. In the latter case, the portions of the signal may be such that their union forms the entire signal, or a continuous part of the signal (i.e. a part of the signal corresponding to a complete time interval, without interruption). Alternatively, certain portions may be disjoint and noncontiguous (in other words, the joining of the portions does not form a continuous part of the signal).

It is noted that the threshold may be dynamic, meaning that it may depend on the portion of the signal considered. In other words, for each portion of the signal, a corresponding threshold may be defined. This makes it possible to adapt as best as possible to variations in the signal, including its low values. As mentioned above, the resting value is believed to have a much greater diagnostic value than the complete signal for certain pathologies, like fibrosis. Using the resting value rather than a value classically determined from a series of measurements taken at random times can therefore reduce the number of overestimations and therefore reduce the number of false positives, and avoid the need for subjects to undergo additional painful and invasive tests, such as biopsies, to rule out a diagnosis of fibrosis. The resting value can therefore be seen as a "corrected" or "modified" value of the measurement of the mechanical property.

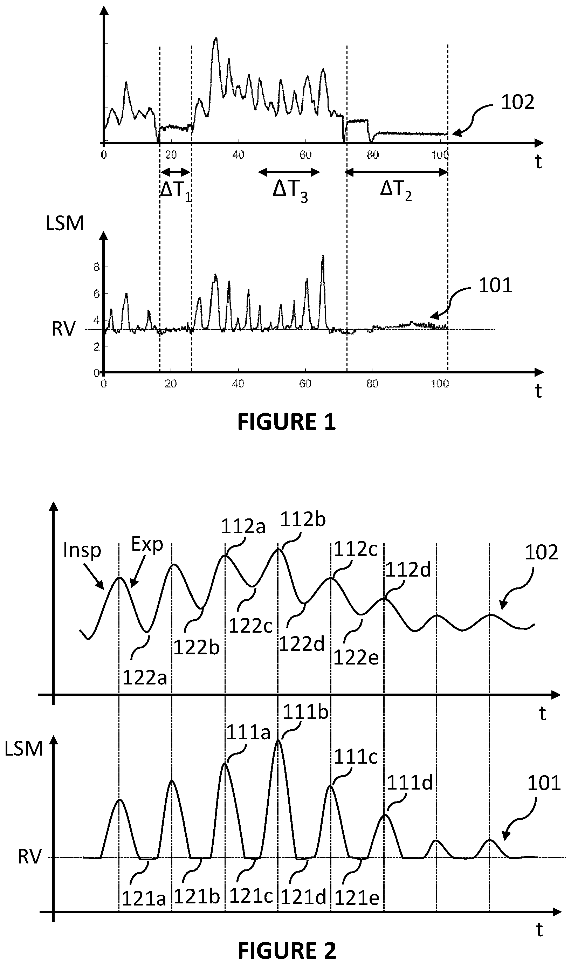

As mentioned above, the way the operator handles the probe plays an important role to obtain high quality results in the stiffness evaluation. As an example, the elastography probe needs to be perpendicular to the skin surface to avoid overestimations. When the probe is not perpendicular to the skin surface, it is rather easy to overestimate liver stiffness by 1 kilopascal (kPa) for example. As a consequence, a low-risk patient with a LSM of 7.5 kPa would potentially be measured at 8.5 kPa which corresponds to intermediate risk according to the AGA guideline 8 kPa threshold (Figure 1 of the article of Kanwal, F et al. "Clinical Care Pathway for the Risk Stratification and Management of Patients With Nonalcoholic Fatty Liver Disease", Gastroenterology 2021, 161 (5), 1657-1669). It is therefore important that stiffness measurement be as precise and reliable as possible.

The method of the invention makes it possible to provide a more reliable measurement of the mechanical property, and thus to limit these overestimations.

It will be appreciated that a novice operator will benefit from the invention as the overestimations due to respiration or probe perpendicularity are automatically removed.

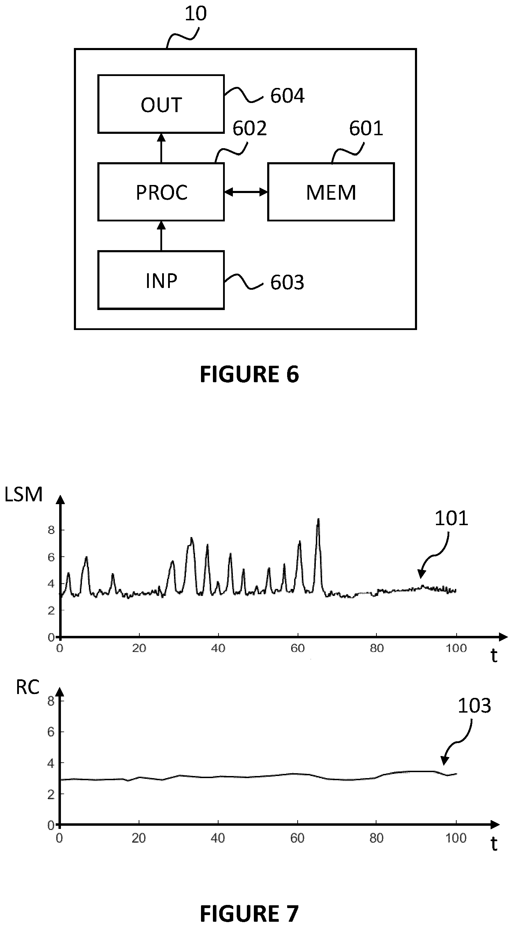

Furthermore, the variability observed on the resting value is lower than the variability of the complete signal which results in better intra and interoperator dependency. In the example of Figure 7, the resting value of liver stiffness is 3.1 ± 0.2 kPa while the average liver stiffness 3.7 ± 0.9 kPa. Not only, this modified value is lower but the variability is significantly reduced.

In one or more embodiments, the elastography method may also comprise:
- determining a dispersion value representing a dispersion of the selected measurements.

For example, this dispersion value can be function of an amplitude, an interquartile range or a standard deviation of the selected measurements.

In one or more embodiments, the determining of the resting value may comprise:
- determining a component of the signal, called resting component, based on the selected measurements; and
- calculating the resting value as a function of a percentile or a mean of the resting component.

Another aspect of the invention relates to an elastography method implemented by computer, comprising:
- acquiring, at a repetition rate of at least 4 measurements per second and for a duration of at least 3 seconds, measurements of a mechanical property of a region of a body of a subject, said region being a part of a liver or a part of a spleen of the subject;
- obtaining a signal representative of the variations of the mechanical property with time, said signal comprising at least part of the measurements of the mechanical property determined;
- in each portion of the signal among at least one portion of the signal, selecting measurements, among the measurements comprised in the portion of the signal, greater than a threshold associated with the portion of the signal; and
- determining a value representative of a variation of the mechanical property with time, called dynamic value, from the selected measurements.

By contrast with the resting value, the dynamic value is associated with variations in the signal which are linked (among other factors) to the respiration of the subject once other factors such as probe perpendicularity are controlled.

The dynamic feature provides information on signal variations, which can be used for example to assess hemodynamic function. The dynamic value may also have an important diagnosis value for cardiovascular disorders or pulmonary diseases, for example.

Also, knowledge of both the resting value and the dynamic value provide a better characterization of the liver parenchyma. Therefore, it is understood that in embodiments, both the resting value and the dynamic value may be determined.

Schematically, it is understood that measurements under the threshold are used to determine the resting value, and measurements above the threshold are used to determine the dynamic value.

In one or more embodiments, the determining of the dynamic value may comprise:
- for each portion of the signal among the at least one portion of the signal, determining a representative value of the portion of the signal, the representative value being function of a mean, a median and/or a standard deviation of the measurements selected in the portion of the signal;
- determining a component, called dynamic component, of the signal based on the representative values determined; and
- determining the dynamic value based on the dynamic component.

For example, the dynamic value may be a function of a percentile or a mean of the dynamic component.

Other embodiments are possible. For example, when a static component is determined, the dynamic component may be determined by subtracting the resting component from the signal.

In one or more embodiments compatible with all the preceding embodiments, in each portion of the signal among the at least one portion of the signal, the threshold associated with the portion of the signal may correspond to a predefined percentage of a difference between a minimum value and a maximum value of the portion of the signal, the predefined percentage being less than 30%.

In alternative embodiments, in each portion of the signal among the at least one portion of the signal, the selected measurements may be lower than or equal to a predetermined percentile of the portion of the signal, the predetermined percentile being comprised between a minimum value and a thirtieth percentile of the signal.

In one or more embodiments, the at least one portion of the signal may comprise a plurality of portions of the signal obtained by applying a sliding window to the signal.

By "sliding window", it is meant a temporal window moving over the data, and therefore defining a portion of the signal comprising the signal values included in this time window. This window moves temporally, thus defining a plurality of consecutive windows corresponding to different time intervals.

A sliding window is defined be two parameters:
- the temporal length of the window, i.e. its temporal size; and
- the sliding interval, which corresponds to the time difference between two consecutive windows.

In the context of the present invention, it is noted that the temporal length and/or the sliding interval are not necessarily fixed values.

For example, in one or more embodiments, the sliding window may comprise a fixed number of measurements.

In such embodiments, all portions of the signal obtained by applying the sliding window to the signal include the same number of measurements.

It is noted that, in one or more embodiments, each measurement may be associated with a respective quality indicator. Each measurement may be considered as "valid" if its quality indicator is above a predefined threshold or indicates that the quality of the measurement is correct. In such embodiments, the sliding window may comprise a fixed number of valid measurements.

Equivalently, the signal can be constructed only from measurements considered as valid (i.e. the signal includes valid measurements and does not include non-valid measurements).

It is understood that in both cases, the spacing between two valid measurements is not fixed (for example, if out of 4 measurements E₁, E₂, E₃, E₄, measurement E₃ is not valid but the other measurements are valid, measurements E₁ and E₂ are spaced by a time which corresponds to 1/Fe, where Fe is the acquisition frequency of measurements, while measurements E₂ and E₄ are spaced by 2/Fe). Thus, if the window includes a fixed number of valid measurements, then the (temporal) length of the window is not necessarily fixed.

In yet other embodiments combinable with the previous one, it is possible to define a minimum threshold of percentage of valid measurements for the sliding window. By "percentage of valid measurements", it is meant the number of valid measurements in a portion of the signal divided by the number of measurements (valid and non-valid) in the portion of the signal. In such embodiments, each portion of the signal includes a percentage of valid measurements above the predefined threshold.

In other embodiments, the sliding window may have a fixed temporal length.

In yet other embodiments combinable with the previous ones, the length of the sliding window may depend on the duration of a respiratory cycle. This duration may be a predefined average duration of a respiratory cycle which is not specific to the subject considered, but which can be associated with one or more categories associated with the subject (for example: age category, sex, Body Mass Index category, etc.). Alternatively, this duration may be a duration specific to the subject, for example an average duration of the respiratory cycle of the subject (determined by using a breathing sensor).

In one or more embodiments, the sliding window may be associated with a sliding interval comprised between 1/fₑ and the temporal length of the sliding window, where fₑ is the repetition rate of the measurements.

Taking for the sliding interval a value less than or equal to the temporal length of the window ensures that the entire signal (or an entire continuous part of the signal) is processed. However, it is possible to use a value of the sliding interval strictly greater than the temporal length of the window. In this case, there is a "gap" between the current window and the next window (and the values of the signal comprised in this gap are not processed).

In one or more embodiments, the sliding interval may be a fixed value, for example comprised between 1/fₑ and 20 seconds, or between 1/fₑ and 10 seconds, or even between 1/fₑ and 5 seconds.

In alternative embodiments, the sliding interval may be defined such that it comprises a fixed number of measurements.

In one or more embodiments, the obtaining of the signal representative of the variations of the mechanical property with time may comprise:
- determining an intermediate signal comprising at least part of the measurements of the mechanical property determined;
- applying to the intermediate signal a smoothing algorithm to obtain the signal representative of the variations of the mechanical property with time.

Applying a smoothing algorithm to the signal makes it possible to "erase" certain local variations in the signal, to capture important patterns of the signal. Such an algorithm is generally used to remove noise from a signal.

Here, the application of such a smoothing algorithm makes it possible to remove part of the overestimations in the signal. Therefore, the resting value and/or the dynamic value can be determined more accurately.

Any smoothing algorithm of the prior art can be used, for example a moving average, a Butterworth filer, an exponential smoothing, a Kalman filter, etc.

Another aspect of the invention relates to an elastography device comprising an electronic unit configured to:
- acquire, at a repetition rate of at least 4 measurements per second and for a duration of at least 3 seconds, measurements of a mechanical property of a region of a body of a subject, said region being a part of a liver or a part of a spleen of the subject;
- obtain a signal representative of the variations of the mechanical property with time, said signal comprising at least part of the measurements of the mechanical property determined;
- in each portion of the signal among at least one portion of the signal, select measurements, among the measurements comprised in the portion of the signal, lower than or equal to a threshold associated with the portion of the signal; and
- determine a value representative of the mechanical property, called resting value, based on the selected measurements.

Yet another aspect of the invention relates to an elastography device comprising an electronic unit configured to:
- acquire, at a repetition rate of at least 4 measurements per second and for a duration of at least 3 seconds, measurements of a mechanical property of a region of a body of a subject, said region being a part of a liver or a part of a spleen of the subject;
- obtain a signal representative of the variations of the mechanical property with time, said signal comprising at least part of the measurements of the mechanical property determined;
- in each portion of the signal among at least one portion of the signal, select measurements, among the measurements comprised in the portion of the signal, greater than a threshold associated with the portion of the signal; and
- determine a value representative of a variation of the mechanical property with time, called dynamic value, from the selected measurements.

It is noted that aspects of the present invention may be implemented as part of any elastography technique, as soon as the elastography device is configured to perform the measurements at sufficient repetition rate.

For example, the above method may be implemented in the context of Transient Elastography techniques, including ARFI (Acoustic Radiation Force Impulse), SWE (Shear Wave Elastography), TE (Transient Elastography) or VCTE (Vibration-Controlled Transient Elastography), provided that the technique makes it possible to obtain measurements at a sufficient rate. Therefore, in one or several embodiments, the elastography device may comprise:
- a probe that comprises: a protruding part to be applied against the body of the subject and at least one ultrasound transducer; wherein, for determining each of the measurements of the mechanical property, the electronic unit may be adapted to:
   - deliver to the body of the subject, via the protruding part, a transient, low frequency mechanical pulse;
   - upon delivery of said mechanical pulse, control the ultrasound transducer to emit a sequence of ultrasound pulses, and acquire echo signals received in response by the ultrasound transducer to track how a low frequency elastic wave induced by the mechanical pulse propagates through the region of the body of the subject;
   - determine the respective measurement of the mechanical property related to low frequency elastic wave propagation.

In particular, the elastography device may be a VCTE device. In this case, the probe may further comprise a low frequency vibrator arranged to move the protruding part of the probe, and the delivery of the transient, low frequency mechanical pulse may comprise:
- control, by the electronic unit, the low frequency vibrator to deliver to the body of the subject said transient, low frequency mechanical pulse.

By "transient pulse", it is meant a mechanical vibration that is temporary. The duration of the pulse, that is the active time, during which there is a substantial motion protruding part (induced by the vibrator in the case of a VCTE device) is followed by a downtime during which there is no or substantially no motion of the protruding part. By substantially no motion, it is meant for instance that, during this downtime, the displacement of the protruding part that may be induced by the vibrator remains smaller than 1/10 or even 1/20 of the peak displacement of protruding part. For the transient pulses mentioned above, an actuation ratio, equal to the pulse's active time, divided by the sum of this active time and the following down time, is typically below 50%, or even below 20%. The downtime is the duration between the end of the active time and a subsequent significant motion of the protruding part (corresponding for instance to a subsequent transient, mechanical pulse), should there be any.

By low frequency pulse, it is meant that the central frequency of the pulse is below 500 Hz, or even below 250 Hz. The central frequency of the pulse is, for instance, the average or the median frequency of the spectrum of the displacement or of the speed of displacement corresponding to that pulse, or the peak frequency of a main peak of this spectrum, or the mean of the -3 dB or -6 dB cutoff frequencies of the spectrum.

In one or more embodiments, when the elastography device is a VCTE device, a peak-to-peak amplitude of a displacement of the protruding part of the probe induced by the low frequency vibrator may be between 0.2 and 10.0 mm, for example between 0.5 and 3.0 mm.

Other features and benefits of the method and apparatus disclosed herein will become apparent from the following description of non-limiting embodiments, with reference to the appended drawings.

### BRIEF DESCRIPTION OF THE DRAWINGS

Aspects of the present invention are illustrated by way of example, and not by way of limitation, in the figures of the accompanying drawings, in which like reference numerals refer to similar elements and in which:
- Figure 1 represents the variations of an LSM signal and a respiratory signal over time;
- Figure 2 schematically represents the influence of respiration on the LSM signal;
- Figure 3 is a flow-chart describing a method for determining a resting component and/or a dynamic component of the stiffness signal according to an embodiment of the invention;
- Figure 4 schematically represents an example of an elastography device according to one or more embodiments of the invention;
- Figure 5 represents a sequence of mechanical and ultrasound pulses emitted by the elastography device of Figure 4 according to one or more embodiments of the invention;
- Figure 6 schematically represents an electronic unit of an elastography device according to one or more embodiments of the invention;
- Figure 7 represents a resting component of the signal obtained according to an embodiment of the invention;
- Figure 8 represents embodiments of the invention, wherein the portions of the signal are obtained by applying a sliding window to the signal;
- Figure 9 represents a dynamic component of the signal obtained according to an embodiment of the invention.

### DETAILED DESCRIPTION

The inventors of the present invention have investigated the influence of respiration on the values of liver stiffness measurement (LSM) or spleen stiffness measurements (SSM).

During the respiratory cycle, abdominal pressure varies. This variation is even greater in the case of abdominal breathing (also called diaphragmatic breathing, as opposed to thoracic breathing). These variations induce changes in the LSM or SSM measurements. To better understand these changes, the inventors simultaneously measured both LSM and respiratory signals.

Figure 1 illustrates the results obtained on one subject. In particular, Figure 1 represents two curves:
- a curve 101 representing the LSM signal; and
- a curve 102 representing the respiratory signal.

The LSM signal 101 was acquired using a VCTE elastography device configured to acquire several liver stiffness measurements per minute, similar to the device presented in patent application EP22305298.6 assigned to the applicant of the present patent application.

Indeed, given that the respiratory frequency of an adult is of the order of 12 to 20 cycles per minute, which means one cycle every 3 to 5 seconds, each cycle being composed of one inspiration and one expiration (in which inspiration occupies approximately one third of the respiratory cycle, and the expiration occupies approximately two thirds of the respiratory cycle), it was interesting to acquire measurements at a sufficiently high rate (at least 4 measurements per second, for example 10 measurements per second) to follow more precisely the variations of the LSM over time, and in particular during a respiratory cycle. The LSM signal 101 is expressed in Kilopascals (kPa).

The respiratory signal 102 was acquired by a respiratory belt measuring changes in chest diameter over time. This signal 102, whose amplitude depends on the subject's chest diameter, is expressed in arbitrary units.

First, it appears from the curves 101, 102 in Figure 1 that LSM values increase during inspiration (i.e. when the respiratory signal 102 is increasing) and decrease during expiration (i.e. when the respiratory signal 102 is decreasing). Thus, the peaks of the LSM signal 101 coincide temporally with the peaks of the respiratory signal 102.

In addition, the value of the peaks (i.e. local maxima) of the LSM signal 101 depends on the volume of inspired air (the "deeper" the inspiration, the higher the value of the LSM peak), while the minimum values of the LSM signal 101 (at the end of respiratory cycle or at the start of respiratory cycle) remains relatively stable.

The relative stability of the minimum values of the LSM signal 101 is all the more remarkable since these minimum values are not affected by the minimum values of the respiratory signal 102. This can be shown in Figure 1, which represents two time intervals ΔT₁, ΔT₂ during which the subject voluntarily held his breath. The first time interval ΔT₁ (around 20-25 seconds) is associated with a first average value of the respiratory signal 102. The second time interval ΔT₂ (around 70-100 s) is associated with a second average value of the respiratory signal 102 lower than the first value (i.e. the average value of the respiratory signal 102 over time interval ΔT₁). It is noted that the "gap" observed in the respiratory signal 102 during the second time interval ΔT₂ (around 80 s) may be caused by a movement of the respiratory belt, or by the fact that the belt has been tightened.

Two observations can be made:
- the temporal variations of the LSM signal 101 are lower during periods of apnea ΔT₁, ΔT₂ (the peaks are lower than when the subject is breathing); and
- the average value of the LSM signal 101 is approximately the same over the two periods of apnea ΔT₁, ΔT₂, and approximately corresponds to the values of the LSM signal at the end or start of a respiratory cycle.

Figure 1 also represents a third time interval ΔT₃ (around 40-55 s) during which the subject is breathing: the respiration curve 102 presents rises and falls corresponding respectively to inspirations and expirations. During this third time interval ΔT₃, the values of local minimums vary quite significantly. In contrast, over this same time interval, the values of the local minimums of the respiratory signal 102 vary very little.

Generally speaking, it can be observed that the minimum values of the LSM signal 102 vary relatively little around an average value RV (represented in Figure 1), independently of variations in the values of the respiratory signal 101. Conversely, the maximum values (i.e. the upward peaks) of the LSM signal 102 vary significantly, and in a manner correlated with the respiratory signal 101.

These observations are represented more schematically in Figure 2.

Figure 2 represents a schematic, simplified version of the curves 101, 102 of Figure 1. As in Figure 1, curve 101 represents the LSM time signal, and curve 102 represents the respiratory time signal.

A phase during which the respiratory signal 102 is increasing corresponds to an inspiration ("Insp" in Figure 2) and a phase during which the respiratory signal 102 is decreasing corresponds to an expiration ("Exp" in Figure 2).

It can be seen in Figure 2 that upward peaks 111a, 111b, 111c, 111d of the LSM signal 101 temporally correspond to upward peaks 112a, 112b, 112c, 112d in the respiratory signal 102. In addition, a deeper respiration (corresponding to a higher upward peak in the respiratory signal 102) is associated with a higher upward peak in the LSM signal 101. This can be seen by comparing upward peaks 111b/112b and 111d/112d for example. The higher upward peak 112b in the respiratory signal 102 follows a deeper inspiration than the lower upward peak 112d. In the LSM signal 101, a higher peak 111b can be observed almost simultaneously with the higher upward peak 112b of the respiratory signal 102, and a lower upward peak 111d can be observed almost simultaneously with the lower upward peak 112d of the respiratory signal 102.

However, whatever the values of the "dips" (i.e. the values of the local minima) 122a, 122b, 122c, 122d, 122e of the respiratory signal 102 (which therefore correspond to ends or beginnings of respiratory cycles), the average values of the low plateaus 121a, 121b, 121c, 121d, 121e remain approximately the same, around a stable value RV. In other words, the values of the dips 122a, 122b, 122c, 122d, 122e of the respiratory signal 102 can vary greatly, but the average values of the corresponding plateaus 121a, 121b, 121c, 121d, 121e of the LSM signal 101 vary very little, around the value RV.

This is a sign that the upward peaks in the LSM signal 101 are mostly influenced by abdominal pressure, while the average value of the local minima (or the average value of the low plateaus) of the LSM signal 101 is probably more correlated with stiffness of the parenchyma (and therefore with a level of fibrosis). The peaks are responsible for overestimations in relation with liver fibrosis. In other words, this average value has probably a much greater preventive or diagnostic value for fibrosis or liver pathologies than the rest of the curve (and therefore than the stiffness indicators currently measured today, calculated as a median or an average of a plurality of stiffness measurements acquired randomly during the respiratory cycle - and therefore potentially during an upward peak of the LSM signal).

From these observations, the inventors of the present invention have devised that the LSM signal 101 can be decomposed into two components:
- a "dynamic" component representative of (a) the influence of pressure variations inside the part of the body for which the stiffness is measured and (b) respiration on the signal; and
- a "resting" component, which is not influenced by these factors, and in particular by respiration. This resting component reflects the more static contribution from the liver parenchyma and is believed to be much more linked to the fibrosis than the dynamic component.

This resting component typically corresponds to a component of the signal around the value RV in Figures 1 and 2.

It is noted that other factors can generate variations in the LSM signal, for example hemodynamic effects. Indeed, in the not yet published patent application EP24305031.7 assigned to the same applicant, the inventors noticed that variations in the cardiac signal (in particular the components of the CVP waveform) can be observed in the LSM signal.

Thus, the dynamic component of the signal reflects or is representative of variations due to respiration as well as the effect of blood pressure due to hemodynamic and cardiovascular characteristics, while the resting component is mainly correlated to fibrosis.

It will be appreciated that the observations above also apply to a spleen stiffness measurement (SSM) signal. Indeed, it is known that the LSM and the SSM are correlated, and that variations in the LSM values are repercussed or reflected in the SSM values. Thus, the variations observed in the LSM signal linked to hemodynamic changes and changes in abdominal pressure are also observable in the SSM signal. Therefore, aspects of the present invention also applies to an SSM signal.

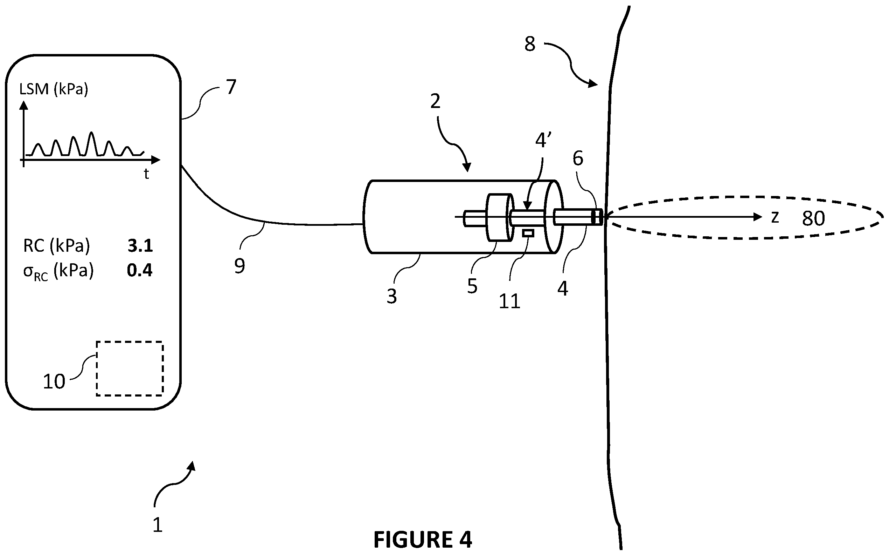

The measurements of the mechanical property may be obtained by using any elastography device. In particular, the measurements of the mechanical property may be acquired by an elastography device using a Vibration-Controlled Transient Elastography (VCTE) technology, such as the device described in Figure 4. It should be noted, however, that aspects of the present invention is not limited to such technology.

The elastography device 1 of Figure 4 comprises a probe 2 including a probe casing 3 (which forms the main body of the probe) to be handheld and a protruding part, which protrudes from the casing 3. The protruding part can be applied against the body 8 of the subject, to deliver mechanical pulses to it, and to transmit and acquire ultrasound (U/S) shots.

In the example of Figure 4, the protruding part is a tip 4, for instance a cylindrical tip (with a circular transducer 6 at its end).

Still, in other embodiments, the protruding part could be an ultrasound head (located at an end of the probe) including an array, for instance a linear array of U/S transducers. In this regard, it may be noted that the proposed technique can be used with a single element ultrasound transducer or with a multi-element ultrasound transducer (like an array of U/S transducers, for example a linear or convex or phased array ultrasound probe). While a single element ultrasound transducer is adapted to display A-mode and M-mode ultrasound imaging, a multi element ultrasound transducer can also display a B-mode image allowing an easier localization of the to-be-measured tissue. In the case of a multi element ultrasound transducer, at least one of the beamformed ultrasound lines is used to track how the mechanical pulses propagate. To this end, using the center beamformed ultrasound line (which is aligned with the probe axis) is beneficial, for symmetry considerations.

The probe 2 comprises also a low frequency vibrator 5, and the U/S transducer 6, which is fixed at an end of a tip 4. Here, the U/S transducer 6 plays both the role of an ultrasound emitter and the role of an ultrasound receiver (alternatively). Still, in other embodiments, the probe may comprise an U/S emitter and an U/S receiver distinct from each other. Here, the U/S transducer 6 is arranged on the axis z of the vibrator. Still, in other embodiments, the U/S transducer could be located elsewhere on the probe, not necessarily on the vibrator's axis.

The tip 4 is actuated by the low frequency vibrator 5. Here, the vibrator 5 is arranged to move the tip 4 relative to the probe casing 3. The vibrator 5 is arranged to move a shaft 4', the end of which forms the tip 4 of the probe. Still, in other embodiments, the probe may be an inertial probe. In such an inertial probe, the tip, or more generally the protruding part of the probe, could be bound to the probe casing with no motion with respect to the probe casing, the vibrator being then arranged to move a mass, inside the casing, to make the whole probe moving towards the tissue and back (by virtue of a recoil effect).

The vibrator 5 is a low frequency vibrator in that it moves the tip with a central, average frequency smaller than 500 hertz, or even smaller than 100 hertz (in contrast with ultrasound shots or echo signals, whose central frequency is typically higher than 1 megahertz, for instance between 1 and 5 megahertz). The vibrator is a low-frequency electro-mechanical actuator, for instance with one or several coils and magnets, like a loud-speaker actuator.

In this device 1, the vibrator 5 is rotationally symmetrical around a vibrator axis, which coincides with the probe axis z. When the vibrator 5 vibrates, it induces displacements that are mainly longitudinal, parallel to its axis. The shaft 4' is centered onto the axis z, and the vibrator 5 is arranged to move this shaft along the axis z.

In practice, the displacement of the ultrasound transducer 6, induced by the vibrator 5, has a peak-to-peak amplitude between 0.2 mm and 10.0 mm, and, in an embodiment, between 0.5 and 3.0 mm.

The probe 2 comprises a displacement sensor 11, arranged to output a measurement signal representative of the displacement of the ultrasound transducer 6. In this embodiment, the measurement signal is representative of the displacement of the ultrasound transducer 6 relative to the probe casing 3. A part of the displacement sensor 11 is fixed on the shaft 4' mentioned above while another part of the sensor is fitted in the probe, with no motion with respect to the casing 3. The displacement sensor 11 may be a Hall-effect sensor, an induction displacement sensor, or any other suitable sensor.

The probe 2 is operatively connected to a central unit 7, which has the structure of a computer (e.g. a laptop, a smartphone, or a dedicated electronic device arranged to control and to interface the probe, and to process the signals acquired). The central unit 7 comprises at least one memory and a processor or processing circuit. The at least one memory is coded with machine readable instructions for carrying out function(s) of the central unit 7 when executed by the processor. The central unit 7 may also comprise a display and a graphical user interface for displaying some pieces of information, such as the curve representing the signal representative of the variations of the mechanical property of the region 80 over time during an examination of the subject, e.g., the resting value and/or the dynamic value,, one or several elastograms, or other pieces of information (e.g. the LSM signal). The display and graphical user interface may be controlled by the processing circuit of the central unit 7 to cause the graphical user interface to display visual information to the operator or prompt the operator of the elastography device 1 to initiate one or more actions or commands. The central unit 7 comprises also at least one user interface, such as a keyboard, a mouse, one or more buttons and/or a touch screen to enter one or more data and/or information and/or parameters. As a non-limiting example, the additional parameters may include the age, height, weight, body mass index (BMI), fat mass index of the patient or subject.

The probe may be connected to the central unit 7 by a connection cable 9, or by a wireless link (e.g. via WiFi or BLUETOOTH^{®}).

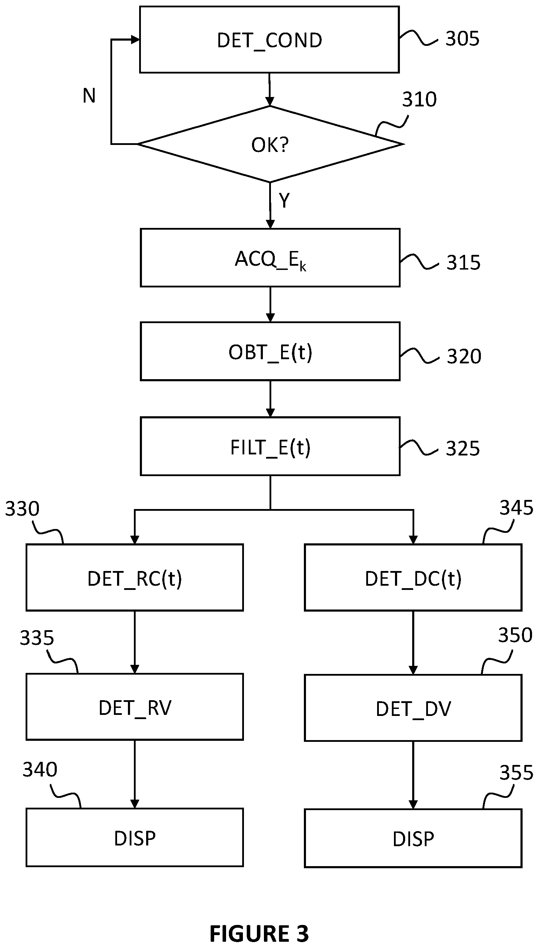

The central unit 7 may also comprise an electronic unit 10 connected to the vibrator 5 and to the U/S transducer 6 and configured (for instance, programmed via instruction stored in a memory) to control the elastography device 1 so that it acquires (or "determines") a plurality of measurements of the mechanical property of the region 80 of the body and implements a method for determining a resting value (or baseline value, or modified value) from the LSM signal according to one or more aspects of the invention (and detailed hereinafter, in reference to Figure 3).

In one or more embodiments, the plurality of measurements of the mechanical property may be acquired in response to a manual triggering by the operator. This manual triggering may be achieved by actuating a pushbutton switch arranged on the probe casing 3, or by actuating a footswitch, for instance. In such a case, when the operator determines, based on one or more pieces of information, that the acquisition of the series of measurements can begin, the operator may trigger acquisition of the measurements. Several measurements of the mechanical property of the probed region, related to low frequency elastic wave propagation (for instance its Young's modulus) are then obtained, each measurement Eₙ being associated with a respective time tₙ, n being an integer greater than 1 (for example, n may be comprised between 10 and 1000). The signal E(t) representative of the variations of the mechanical property over time may then be obtained from samples Eₙ = E(tₙ). The one or more pieces of information on the basis of which the operator determines that he can trigger the acquisition of the series of measurements may include, for example, information that the position and direction of the probe 2 is adequate (as detailed for instance in the US patent application US17/695,053 assigned to the applicant) and/or information that the tip 4 is applied against the body 8 of the subject (for example, based on contact force level measured by a force sensor - like a strain gauge - or deduced from the position of the shaft 4' pushed into the casing when the tip is pressed on the subject's body).

It is noted that, in these embodiments, it is the emission of the first mechanical pulse associated with the series of measurements which is triggered manually by the operator. The following mechanical pulses are delivered automatically at a predefined repetition rate of at least 4 pulses per second, and for a predefined duration of at least 3 seconds (for example at least 6 seconds or at least 10 seconds).

In alternative embodiments, even the first mechanical pulse associated with the series of measurements is automatically emitted, based on one or more pieces of information. For example, the electronic unit 10 may be configured to determine whether one or more conditions are satisfied and to trigger the emission of the series of mechanical pulses when all necessary conditions are satisfied. For example, the electronic unit 10 may be configured to determine whether the position and direction of the probe 2 are adequate and/or whether the tip 4 is applied against the body 8 of the subject, and if these conditions are met, the electronic unit 10 may trigger the emission of the series of mechanical pulses.

In these embodiments, the determination, by the electronic unit 10, of whether the one or more conditions are satisfied may be performed in response to a manual triggering by the operator, achieved for instance via the touch screen of the central unit 7.

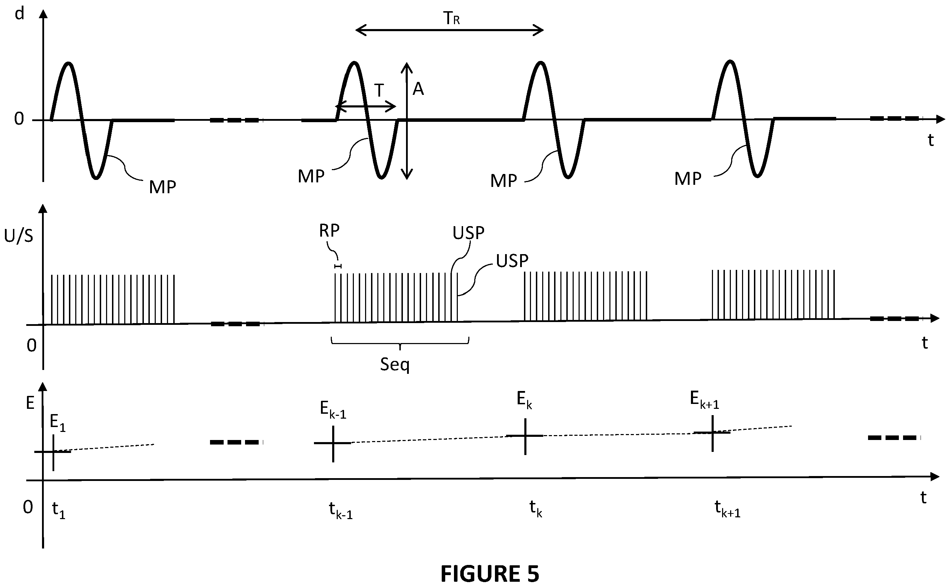

As represented in Figure 5, to obtain the series of measurements of the mechanical property, the electronic unit 10 may be configured to control the low frequency vibrator 5 to deliver to the body 8 of the subject, successively and repeatedly, a plurality of pulses MP (referred to as "mechanical pulses"), each pulse MP being a transient, low frequency mechanical pulse. Each mechanical pulse MP corresponds to a transient displacement d of the shaft 4' along the axis z directed towards the subject's body (see Figure 4). The displacement d of the shaft 4' induced by the vibrator may for instance correspond to one period of a sinusoid having a duration T between 5 ms and 50 ms (i.e. a frequency between 20 Hz and 200 Hz).

The mechanical pulses MP are typically delivered with a predefined repetition rate of at least 4 pulses per second, for instance a repetition rate of 5 or even 10 pulses per second or more. So, the mechanical pulses MP are repeated with a repetition period T_{R} which is quite short, typically below 0.25 second (corresponding to a frequency above 4 Hz). It will be appreciated that the shorter the repetition period T_{R}, the closer the measurements of the mechanical property can be made, and the more accurate the temporal signal constructed from these measurements, which makes it possible to properly monitor the variations of the value of the mechanical property over time. On the other hand, the shorter the repetition period T_{R}, the greater the computational resources required to process the data received, which may require specific arrangements of the elastography device described previously (for example, by using the pre-compensation technique presented in patent application EP22305298.6 assigned to the same applicant).

The displacement d of the shaft 4', induced by the vibrator 5, has a peak-to-peak amplitude A between 0.2 mm and 10.0 mm, and in an embodiment between 0.5 and 3.0 mm.

The central frequency of each mechanical pulse MP may be between 10 Hz and 500 Hz, for example between 50 Hz and 200 Hz when the elastography device is configured to characterize the liver of patients (typically 50 Hz in this case).

As represented in Figure 5, for each mechanical pulse MP, the electronic unit 10 controls the U/S (ultrasound) transducer 6 (with the U/S pulser 41 of the U/S front end 40, among others) so that the U/S transducer 6 emits a sequence Seq of ultrasound pulses USP, and acquires echo signals received in response by the U/S transducer 6, to track how the mechanical pulse MP propagates through the probed region 80 of the body 8 of the subject, located in front of the tip 4 of the probe.

For this sequence Seq, the central frequency of each ultrasound pulse USP is comprised for instance between 0.5 and 10.0 megahertz. The ultrasound pulses of the sequence Seq may be transmitted one at a time, two successive pulses being separated by a pulse repetition period RP, this pulse repetition period being typically between 0.1 millisecond and 2 milliseconds (which corresponds to a pulse repetition frequency between 0.5 kilohertz and 10 kilohertz), and in an embodiment between 0.3 ms and 1 ms. The ultrasound pulses USP of the sequence Seq mentioned above may also be transmitted by groups, for instance by groups of two pulses (to compute correlations between the two corresponding echo signals). The two pulses of each group may be separated by duration between 50 and 200 microseconds, while the groups of pulses themselves are separated by a longer duration, for instance higher than 0.2 or 0.5 ms. It will be appreciated that other transmission sequences can also be considered in various embodiments. Regarding the total duration of the sequence of U/S pulses Seq, it may be between 25 ms and 200 ms. This duration may be selected depending on the shear wave frequency and depending on the speed of propagation of the elastic wave which is the slower and depending on the depth of the region to be observed. For instance, for a shear wave frequency of 50 Hz, an 80 mm depth and a speed of propagation of 1 m/s (typical for shear waves in the liver of a subject), the sequence may have a duration of 100 ms.

Regarding the echo signals, acquired to track the propagation of the mechanical pulse considered, each of them is formed by a signal, received over time t by the U/S transducer 6 after the emission of one of the U/S pulses USP. It is more precisely the signal received within a given temporal window starting after this emission and having a given duration.

In the embodiments described herein, for each mechanical pulse MP, the electronic unit 10 determines tissue strain data, representative of tissue strain within the region 80, as a function of time t and as a function of depth z within the region 80. The tissue strain data is determined from the echo signals acquired to track how the mechanical pulse MP propagates through the region 80. When represented graphically as a function of time and depth, such tissue strain data forms an elastogram, from which a measurement of the mechanical property (e.g. the tissue stiffness) may be determined.

The tissue strain data is determined, from the echo signals, using a correlation technique or another patterning matching algorithm, to determine how portions of the tissue move under the influence of the elastic wave that is passing through it (the elastic wave being generated by the periodic mechanical vibration delivered by the system). For instance, for each couple of two successively received echo signals, the two echo signals are correlated with each other, which enables one to determine tissue displacement (namely, the tissue displacement that occurred between the two U/S pulses), as a function of depth, and at given time.

As mentioned above, for each mechanical pulse MP, or at least for several of them, respective tissue strain data may be obtained and a respective measurement (E₁, Eₖ₋₁, Eₖ, Eₖ₊₁ in Figure 5) of the mechanical property of the region 80 may be determined. Each determined measurement Eₙ may be associated with a respective time tₙ which represents the instant at which the measurement is performed. For example, as in the embodiment represented in Figure 5, each measurement Eₙ is associated with a respective time tₙ equal to the time at which the corresponding mechanical pulse MP has been emitted. Other embodiments are possible. For example, each measurement Eₙ may be associated with a respective time tₙ equal to the time at which the last of the echo signals is acquired in response to the emission of the corresponding sequence Seq of U/S pulses USP. Alternatively, each measurement Eₙ may be associated with a respective time tₙ equal to the time at which the measurement Eₙ is determined (i.e. after the determination of the corresponding tissue strain data). It is noted that the values of the times tn obtained by all these embodiments differ very little from each other, and that this difference has little impact on the interpretation of the signal E(t) obtained.

All the determined measurements E₁, Eₖ₋₁, Eₖ, Eₖ₊₁ correspond to discrete samples of a signal E(t) representative of the variations of the value of the mechanical property of the region 80 over time. The signal E(t) may be a discrete signal including only the determined measurements E₁, Eₖ₋₁, Eₖ, Eₖ₊₁, or it may be a continuous signal obtained from samples E₁, Eₖ₋₁, Eₖ, Eₖ₊₁, for example by using linear interpolation (see the curve in dashed line of Figure 5) or spline interpolation.

In embodiments, each measurement is associated with a respective quality indicator indicating a quality of the measurement. For example, the quality indicator may be a binary variable (e.g. "non-valid / valid") or an ordinal variable (qualitative or semi-quantitative) having more than two possible values. In the case of an ordinal variable, it is possible to associate each measurement with a "non-valid" or "valid" status from the quality indicator of the measurement. In embodiments, the signal may include only measurements being determined as "valid" (the "non-valid" measurements being therefore discarded). The status of each measurement (valid or non-valid) may be stored, to be used for processing the signal.

The mechanical property of the tissue, related to low frequency shear wave propagation may be a quantity related to the tissue stiffness, such as the propagation speed of shear waves Vₛ, the shear modulus of the tissue or the Young's modulus E of the tissue (which can be derived from the slope of the stripes identified in the elastogram, or from the variation of the time of flight of the measurement pulse as a function of depth). It may also be a quantity related to low frequency shear wave attenuation in the tissue, like viscosity.

As mentioned above, it is noted that, even the elastography device represented in Figure 4 is a VCTE device, aspects of the present invention can be generalized to any type of elastography device making it possible to obtain measurements with a sufficiently high repetition rate (e.g. at least 4 measurements per second). In particular, aspects of the present invention may be applied to other TE (Transient Elastography) devices, for example devices implementing ARFI (Acoustic Radiation Force Impulse) or SWE (Shear Wave Elastography) technologies. In ARFI and SWE, unlike in the VCTE technology, a longitudinally moving shear wave is not generated by an external vibrator. Instead, in ARFI, pressure radiation generated by ultrasounds is focused in a region of interest. The radiation pressure generates a laterally moving shear wave that is tracked outside the region of interest using ultrasound tracking pulses.

As mentioned above, an aspect of the invention relates to a method for extracting the resting component and/or the dynamic component from the signal representative of temporal variations of the mechanical property, in an embodiment of the invention. A flow-chart describing an embodiment of such a method is represented in Figure 3. The method may be carried out by the elastography device or by an external device in communication with the elastography device. In the latter case, the external device is configured to receive the measurements or the signal from the elastography device and to implement the method of Figure 3.

In the following, the mechanical property corresponds to the tissue stiffness, and more specifically a liver stiffness. It will be appreciated that any other mechanical property may be measured, for instance the elasticity, the Young's modulus, the shear modulus, the shear wave speed, the viscoelasticity, the viscosity or any composite biomarker deriving from (or combining) one or more of the previous physical quantities. Also, it will be appreciated that other parts of the body may be considered, in particular the spleen.

In an optional step 305, one or more conditions for triggering an acquisition of liver stiffness measurements Eₖ (with k an integer greater than 1) of the tissue stiffness (or simply "stiffness") may be determined. Such conditions may include, for example:
- a condition relative to whether the position and direction of the probe 2 is adequate, i.e. whether the probe 2 is correctly positioned with respect to the region 80 of the body 8 for which measurements of the mechanical property are to be obtained. The US patent application US17/695,053 assigned to the applicant details examples of determination of such condition; and/or
- a condition relative to whether the tip 4 is properly applied against the body 8 of the subject. Such condition may be determined, for example, based on contact force level measured by a force sensor (like a strain gauge) or deduced from the position of the shaft 4' pushed into the casing when the tip is pressed on the subject's body.

In an optional step 310, the electronic unit 10 may determine whether all conditions determined at step 305 are met. When at least one condition is not met (step 310, arrow "N"), the acquisition of the measurements Eₖ does not begin. When all conditions are met (step 310, arrow "Y"), the acquisition of the measurements Eₖ can begin (step 315). In one or more embodiments, the acquisition of the measurements Eₖ may be performed semi-automatically (for example, the operator is informed that all conditions are met - e.g. using information displayed on the screen or a visual indication on the probe - and push a button to trigger the acquisition of the first measurements, all the subsequent measurements being automatically acquired), or may be fully automated (for example, as soon as the electronic unit determines that all conditions are met, the series of mechanical pulses are emitted and the corresponding measurements Eₖ are determined).

In step 315, a plurality of measurements of a mechanical property of a region of a liver or a spleen of a subject are acquired at a high repetition rate (at least 4 measurements per second), and during a time of at least 3 seconds (which is an average minimum duration of a respiration cycle of an adult), and in one or more embodiments more than 6 seconds, to be able to capture variations of the mechanical property over one complete respiration cycle and possibly two or more respiration cycles. In practice, a repetition rate of at least 4 measurements per second makes it possible to obtain a resulting signal that is sufficiently precise to observe and extract a resting component and, eventually, a dynamic component from the signal. It will be appreciated that this repetition rate can be higher, for example 5, 6, 7, 8, 9, 10 or more than 10 measurements per second.

As mentioned above, the measurements Eₖ of the mechanical property can be obtained with a Vibration-Controlled Transient Elastography (VCTE) device, as the elastography device 1 described above with reference to Figure 4. However, aspects of the present invention can apply to other elastography techniques, including elastography techniques in which shear wave can be generated by other means. For example, an aspect of the invention is compatible with shear wave generation using acoustic radiation force. Acoustic radiation force is a technique in which the shear wave is generated using long burst (several tens of microseconds) of ultrasound signals. It does not require the use of a vibrator located in the probe. Generally speaking, any elastography technique may be used as soon as it allows the measurements to be acquired at a sufficiently high rate (at least 4 measurements per second).

Also, at step 315, the measurements Eₖ are acquired over a duration covering at least one respiration cycle (whose duration is, in general, between 3 and 5 seconds). Therefore, the duration is at least 3 seconds, for example at least 5 seconds. In addition, in order to cover at least two respiratory cycles, this duration can be chosen greater than 6 seconds, even greater than 10 seconds or even greater than a few tens of seconds (for example, between 10 and 120 seconds).

As mentioned above, each measurement Eₖ is associated with a respective time tₖ, which may be for example the time at which the measurement is performed, or, when the measurement is obtained from an emission of a mechanical pulse, the respective time associated with the measurement may be a time at which the corresponding mechanical pulse is delivered. Other embodiments are possible.

A signal E(t) is then obtained (step 320) from the measurements Eₖ determined at step 315. The signal E(t) may comprise all the measurements acquired or several measurements among all the measurements acquired. For example, in embodiments, each measurement may be associated with a quality indicator or a validity indicator, and the signal E(t) may only include the measurements whose quality indicator exceeds a predetermined threshold or whose validity indicator indicates that the measurement is valid.

The stiffness signal E(t) may be discrete or continuous. For example, the stiffness signal E(t) may be a discrete signal comprising all or part of the measurements acquired: E(t) = {E₁, E₂, ...}. Alternatively, the stiffness signal E(t) may be a continuous signal such that E(tₖ)=Eₖ (k being an integer), wherein the values of the signal between two instants tₖ, tₖ₊₁ associated with two consecutive measurements Eₖ, Eₖ₊₁ are obtained by using an interpolation (for example a linear interpolation or a spline interpolation).

Optionally, at step 320, a curve representative of at least a portion of the signal E(t) may be displayed on a screen. Such display can provide important diagnostic information to the medical practitioner in charge of the subject.

In an optional step 325, the obtained signal E(t) may be smoothed to remove or attenuate local variations of the signal. For example, a technique of exponential smoothing (also called exponential moving average - EMA), or a Kalman filter, or any other smoothing algorithm of the prior art, may be applied to signal E(t). Such filtering makes it possible to reduce the variability of the LSM due other factors than the respiration, for example due to hemodynamic effects or abdominal pressure changes.

In one or more embodiments, a resting component is extracted from the stiffness signal E(t) at step 330. As mentioned above, this resting component corresponds to variations in the stiffness signal E(t) which are not due to the respiration of the subject. As presented above with reference to Figure 1, this resting component corresponds comprises "low" values of the signal, for example portions of the signal during which the subject holds his breath or portions of the signal corresponding to an end or a beginning of a respiratory cycle. It is noted that these portions still comprise variations due to factors other than respiration, in particular hemodynamic changes.

During step 330, a set of measurements, among all the measurements Eₖ included in the signal E(t), is selected and the resting component RC(t) includes the selected measurements. The selected measurements are "low" values of the signal, which can be determined by selecting, for each portion of the signal among at least one portion of the signal E(t), only measurements which are below a threshold associated to the portion of the signal considered (in other words, the threshold may vary from one portion to another).

The at least one portion of the signal may include one portion of the signal (which can be the complete signal) or several portions of the signal. In this case, the portions of the signal may or may not overlap.

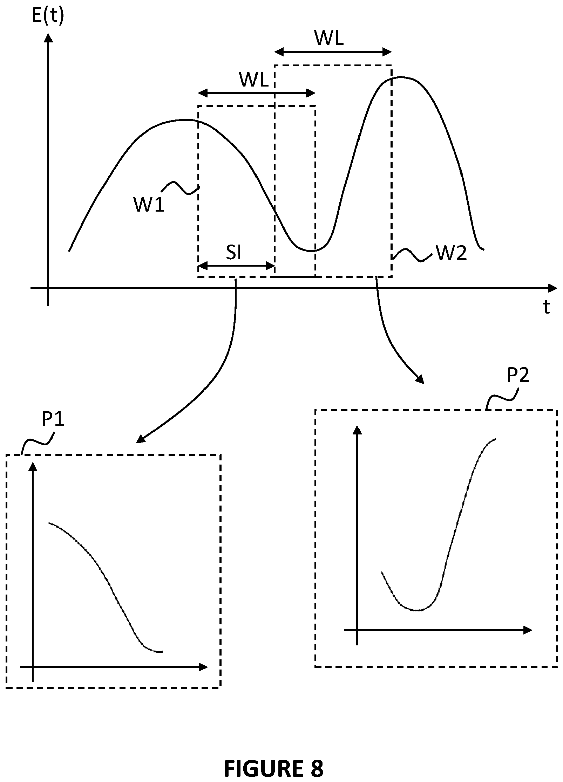

In embodiments, portions of the signal are obtained using a sliding window applied to the signal. Such embodiments are represented for example in Figure 8.

Figure 8 represents an example of a curve of the stiffness signal E(t), and two consecutive positions W1, W2 of the sliding window. In Figure 8, the stiffness signal is continuous, but this is not mandatory. Each position W1, W2 of the sliding window defines a corresponding portion P1, P2 of the signal.

The length of the sliding window is noted WL and corresponds to the temporal size of the window. In the context of the present invention, it is noted that the length WL of the sliding window can be fixed, or not. For example, the length WL of the sliding window can be such that the portions P1, P2 of the signal obtained all comprise the same number of measurements (the measurements acquired in step 315). As mentioned above, in embodiments, the stiffness signal E(t) is built only from a part of measurements. In other words, in these embodiments, certain measurements are not included in the stiffness signal E(t). Therefore, the time elapsed between two measurements of the signal E(t) is not necessarily fixed and can vary between two pairs of measurements. In this case, if the size of the sliding window is defined such that each portion of the signal includes a fixed number of measurements, it is understood that the length WL of the sliding window (i.e. the time interval associated with the portion of the signal thus obtained) can vary.

For example, the length WL of the sliding window can be defined such that each corresponding portion of the signal comprises a fixed number N of measurements, with N an integer greater than 1 (e.g. comprised between 10 and 100). If all measurements are kept in the stiffness signal E(t), the length WL of the sliding window is fixed and equal to N/Fe, where Fe is the acquisition frequency, i.e. the frequency at which the measurements are obtained, i.e. Fe = 1/Te, where Te represents the time between two consecutive measurements. If certain measurements are not kept in the signal, and the sliding window includes a fixed number N of measurements, then the length WL of the sliding window is variable, and takes values between N/Fe and N/(Fe×Rₘᵢₙ), where Rₘᵢₙ is the minimum rate of kept measurements (i.e. the minimum rate of measurements kept to construct the signal over all windows).

Alternatively, the length WL of the sliding window can be fixed, and comprised, for example, between 3 s and 20 s, and more particularly between 5 s and 15 s.

Still referring to Figure 8, the sliding interval SI of the sliding window corresponds to the time interval between the beginning of a window and the beginning of the next window (or, equivalently, between the start times of two consecutive signal portions P1, P2).

The sliding interval is greater than or equal to 1/Fe to include, in the next signal portion P2, at least one new measurement compared to the previous signal portion P1 (if this measurement was not discarded during the construction of the signal E(t)).

In some embodiments, the sliding interval SI is lower than or equal to the length WL of the sliding window. In this case, two consecutive windows partially overlap, or directly follow each other (but there is no "gap" between two consecutive windows). This feature is not mandatory, but it allows the entire signal E(t) to be covered.

In the context of the present invention, it is noted that the sliding interval SI can be fixed or not.

For example, the sliding interval SI can be defined such that it comprises a fixed number K of measurements, with K an integer greater than or equal to 1 (e.g. comprised between 1 and 50). In particular, the integer K can be determined as a function of N. For example, K can be an integer such that: a×N - 1 < K ≤ a×N, or such that a×N ≤ K < a×N + 1 (i.e. K can be the floor or the ceiling of a×N), a being a real number such that 0 < a ≤ 1 (for example, a = 0.5).

Alternatively, the sliding interval SI can be fixed, for example comprised between 1/Fe and 20 s.

Referring again to Figure 3, during step 330, a component, called rest component RC(t), of the signal E(t) is constructed from "low" values of the signal E(t). Examples of such construction are provided below, after the description of Figure 3.

Then, at step 335, a value, called resting value RV is determined from the resting component RC(t). For example, the resting value RV can be function of a standard deviation and/or a mean and/or one or more percentiles of the resting component RC(t). In particular, the resting value RV may be the mean of the resting component RC(t), or the median of the resting component RC(t), or the i-th percentile of the resting component RC(t), with i comprised between 0 and 20, for example i = 5 or 10. Other functions may be used.

The resting value RV and/or the resting component RC(t) may optionally be displayed at step 340.

In one or more embodiments, another component of the signal E(t), called dynamic component DC(t) is extracted from the signal E(t), at step 345. The dynamics component reflects variations, in the stiffness signal E(t), which are due (among other factors) to the breathing of the subject.

It is noted that according to embodiments, the resting component and/or the dynamic component may be extracted from the signal E(t).

In one or more embodiments, the dynamic component DC(t) is determined from a set of measurements, among all the measurements Eₖ included in the signal E(t), the set of measurements being determined by selecting, for each portion of the signal among at least one portion of the signal E(t), only measurements which are above a threshold associated to the portion of the signal considered (in other words, the threshold may vary from one portion to another). It is noted that the threshold may be defined in the same manner as in step 330, or not. For example, two different percentile values can be used to define the threshold (for example, the 5th or 10th percentile for the resting component, and the 20th or 30th percentile for the dynamic component).

In one or more embodiments, the dynamic component DC(t) is determined based on measurements Eₖ that were not used to determine the resting component RC(t). In this case, it is understood that the threshold is defined similarly for step 330 and for step 345.

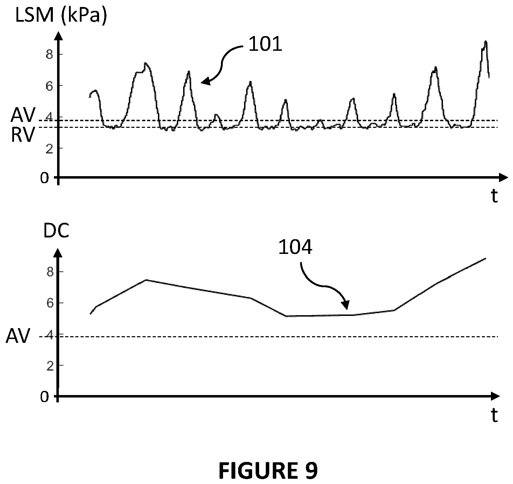

In one or more embodiments, the dynamic component DC(t) is a function of a subtraction between the stiffness signal E(t) and the resting component: DC(t) = F[E(t) - RC(t)], where F is a function. For example, DC(t) may be the mean or the median of the difference (E(t) - RC(t)). In the example represented in Figure 9, the curve 104 of the dynamic component DC(t) corresponds to the mean of the difference (E(t) - RC(t)), where the resting component RC(t) is calculated as is in the example of Figure 7 detailed hereinafter. Figure 9 also represents the average stiffness value AV of the LSM signal 101. It can be observed in the top graph of Figure 9 that this single value AV is greater than the resting value RV determined according to an aspect of the present invention. Thus, taking the average value of the signal as a representative value of the measurement of liver elasticity can lead to diagnostic errors or to classify a subject in a higher risk category (and therefore require moving to the subject to invasive and potentially unnecessary additional examinations).

In other embodiments, the dynamic component DC(t) is a function of a subtraction between the stiffness signal E(t) and the resting value RV: DC(t) = G[E(t) - RV], where G is a function. For example, DC(t) may be obtained by subtracting the resting value RV from the mean or the median of the signal E(t).

Then, at an optional step 350, a dynamic value DV may be determined from the dynamic component DC(t). For example, the dynamic value DV can be function of a standard deviation and/or a mean and/or one or more percentiles of the dynamic component DC(t). In particular, the dynamic value DV may be the mean or the median of the dynamic component DC(t), the standard deviation of the dynamic component DC(t), or the amplitude (difference between the maximum value and the minimum value) of the dynamic component DC(t).

The dynamic component DC(t) and/or the dynamic value DV may optionally be displayed at step 355.

Examples of calculations of the resting component RC(t) are now described. In these examples, the signal comprises a plurality of signal portions (which may partially overlap) obtained by applying a sliding window to the signal E(t). It is understood that there could be a single portion (the complete signal) but considering several portions of the signal E(t) makes it possible to take into account the temporal evolution of the signal E(t), and its "low" values.

It is noted that the calculations below can be applied to the signal E(t) obtained in step 320 of Figure 3 or, when a filtering step 325 is implemented, to the filtered signal. For the sake of simplification, reference is made below to the signal E(t), even when it concerns the filtered signal.

In a first example, for each portion P1, P2 of the signal E(t) among the plurality of portions of the signal E(t), only values less than or equal to a threshold, which corresponds to the value of the 5th percentile of the portion of the signal considered, are selected. When the signal E(t) is a discrete signal comprising a plurality of measurements Eₖ, only measurements less than or equal to a threshold, which corresponds to the value of the 5th percentile of the series of measurements comprised in the portion of the signal considered, are selected.

The resting component RC(t) is then determined based on the selected measurements. For example, the resting component RC(t) may be a discrete function of time comprising the selected measurements. Alternatively, the resting component RC(t) may be a continuous function of time comprising the selected measurements and "completed" by an interpolation. In embodiments, a processing may be applied to the obtained signal, for example a smoothing algorithm.

An example of a resting component RC(t) obtained by using the calculation technique of this first example is represented in Figure 7. More specifically, Figure 7 represents two curves:
- the curve 101 of Figure 1 representing the LSM signal (expressed in kPa); and
- a curve 103 representing the resting component RC(t) (also expressed in kPa).

In this example, the resting component is a function which varies very little around a value approximately equal to 2.9 kPa (which can be taken as the resting value).

It will be appreciated that the example above can be extended to any percentile, for example the i-th percentile, with i an integer between 0 and 20, for example between 5 and 10. It is understood that when i = 0, this amounts to selecting the minimum values on each portion of the signal.

It is noted that the threshold corresponds to the i-th percentile of the portion of the signal, and therefore depends on the portion of the signal on which it is determined. In other words, each portion of the signal is associated with a respective threshold.

In a second example, for each portion P1, P2 of the signal E(t) among the plurality of portions of the signal E(t), only values less than or equal to a threshold, which corresponds to a predefined percentage of the amplitude of the portion of the signal considered, are selected. The amplitude of the portion of the signal corresponds to the difference between the maximum value (or measurement) and the minimum value (or measurement) of the portion of the signal, the percentage being comprised between 0 and 20 %. In other words, the threshold Th(Pk) associated with portion Pk of the signal is: Th(Pk) = p × [Max(Pk) - Min(Pk)], where Max(Pk) denotes the maximum value (or measurement) of the portion Pk, min(Pk) denotes the minimum value (or measurement) of the portion Pk and p is such that 0 < p ≤ 0.20 (for example, 0 < p ≤ 0.10, e.g. p = 0.05).

The resting component RC(t) is then determined based on the selected measurements. For example, the resting component RC(t) may be a discrete function of time comprising the selected measurements. Alternatively, the resting component RC(t) may be a continuous function of time comprising the selected measurements and "completed" by an interpolation. In embodiments, a processing may be applied to the obtained signal, for example a smoothing algorithm.

It is noted that the threshold is calculated, in this second example, from the amplitude of the portion of the signal, and therefore depends on the portion of the signal on which it is determined. In other words, as in the first example, each portion of the signal is associated with a respective threshold.

It will be appreciated that the preceding examples are in no way limiting, and it is understood that any signal processing method making it possible to select low values of this signal can be used.

Figure 6 schematically represents an electronic unit of an elastography device 1 configured to implement a method for extracting a resting component and/or a dynamic component from the stiffness signal, according to one or more embodiments of the invention.

In this embodiment, the electronic unit 10 can form at least part of a computer, and includes a non-transitory memory 601 to store program instructions loadable into a circuit computer 602 (e.g. a microelectronic circuit or circuitry) and adapted to cause the computer circuit 602 to carry out one or several steps of the method(s) or function(s) of the device(s) described herein when the program instructions are executed by the computer circuit 602.

The memory 601 may also store data and useful information for carrying the steps of the method(s) described herein. The circuit 602 may be for instance:
- a processor or a processing unit adapted to interpret instructions in a computer language, the processor or the processing unit may comprise, may be associated with or be attached to a memory comprising the instructions, or
- the association of a processor / processing unit and a memory, the processor or the processing unit adapted to interpret instructions in a computer language, the memory comprising said instructions, or
- an electronic card wherein the steps of the invention are described within silicon, or
- a programmable electronic chip such as a FPGA chip (for « Field-Programmable Gate Array »).

This electronic unit 10 further comprises an input interface 603 for receiving values of the LS measurements and an output interface 604 for providing at least the resting component and/or the dynamic component.

Optionally, the computer circuit 602 may be connected to a screen and may be configured to control the screen and the graphical user interface to cause the graphical user interface to display predetermined information such as, for example, the curve of the LSM signal, the curve of the resting component, the value of the resting value, etc.

A person skilled in the art will readily appreciate that various parameters disclosed in the description may be modified and that various embodiments disclosed may be combined without departing from the scope of the invention.

For example, various aspects of the present disclosure may be used alone, in combination, or in a variety of arrangements not specifically described in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Having described above several aspects of at least one embodiment, it is to be appreciated various alterations, modifications, and improvements will readily occur to those skilled in the art. Such alterations, modifications, and improvements are intended to be aspects of this disclosure. Accordingly, the foregoing description and drawings are by way of example only.

## Claims

1. An elastography method implemented by computer comprising:
- acquiring, at a repetition rate of at least 4 measurements per second and for a duration of at least 3 seconds, measurements of a mechanical property of a region of a body of a subject, said region being a part of a liver or a part of a spleen of the subject;
- obtaining a signal representative of the variations of the mechanical property with time, said signal comprising at least part of the measurements of the mechanical property determined;
- in each portion of the signal among at least one portion of the signal, selecting measurements, among the measurements comprised in the portion of the signal, lower than or equal to a threshold associated with the portion of the signal; and
- determining a value representative of the mechanical property, called resting value, based on the selected measurements.

2. The elastography method of claim 1, wherein the determining of the resting value comprises:
- determining a component of the signal, called resting component, based on the selected measurements; and
- calculating the resting value as a function of a percentile or a mean of the resting component.

3. An elastography method implemented by computer comprising:
- acquiring, at a repetition rate of at least 4 measurements per second and for a duration of at least 3 seconds, measurements of a mechanical property of a region of a body of a subject, said region being a part of a liver or a part of a spleen of the subject;
- obtaining a signal representative of the variations of the mechanical property with time, said signal comprising at least part of the measurements of the mechanical property determined;
- in each portion of the signal among at least one portion of the signal, selecting measurements, among the measurements comprised in the portion of the signal, greater than a threshold associated with the portion of the signal; and
- determining a value representative of a variation of the mechanical property with time, called dynamic value, from the selected measurements.

4. The elastography method of claim 3, wherein the determining of the dynamic value comprises:
- for each portion of the signal among the at least one portion of the signal, determining a representative value of the portion of the signal, the representative value being function of a mean, a median and/or a standard deviation of the measurements selected in the portion of the signal;
- determining a component, called dynamic component, of the signal based on the representative values determined; and
- determining the dynamic value based on the dynamic component.

5. The elastography method of claim 4, wherein the dynamic value is a function of a percentile or a mean of the dynamic component.

6. The elastography method of any one of claims 1 to 5, wherein, in each portion of the signal among the at least one portion of the signal, the threshold associated with the portion of the signal corresponds to a predefined percentage of a difference between a minimum value and a maximum value of the portion of the signal, the predefined percentage being less than 30%.

7. The elastography method of any one of claims 1 to 5, wherein, in each portion of the signal among the at least one portion of the signal, the threshold associated with the portion of the signal corresponds to a predetermined percentile of the portion of the signal, the predetermined percentile being comprised between a minimum value and a thirtieth percentile of the signal.

8. The elastography method of any one of claims 1 to 7, wherein the at least one portion of the signal comprises a plurality of portions of the signal obtained by applying a sliding window to the signal.

9. The elastography method of claim 8, wherein the sliding window comprises a fixed number of measurements.

10. The elastography method of claim 8, wherein the sliding window has a fixed temporal length.

11. The elastography method of any one of claims 8 to 10, wherein the sliding window is associated with a sliding interval comprised between 1/fₑ and the temporal length of the sliding window, where fₑ is the repetition rate of the measurements.

12. The elastography method of any one of the preceding claims, wherein the obtaining of the signal representative of the variations of the mechanical property with time comprises:
- determining an intermediate signal comprising at least part of the measurements of the mechanical property determined; and
- applying to the intermediate signal a smoothing algorithm to obtain the signal representative of the variations of the mechanical property with time.

13. An elastography device comprising an electronic unit configured to:
- acquire, at a repetition rate of at least 4 measurements per second and for a duration of at least 3 seconds, measurements of a mechanical property of a region of a body of a subject, said region being a part of a liver or a part of a spleen of the subject;
- obtain a signal representative of the variations of the mechanical property with time, said signal comprising at least part of the measurements of the mechanical property determined;
- in each portion of the signal among at least one portion of the signal, select measurements, among the measurements comprised in the portion of the signal, lower than or equal to a threshold associated with the portion of the signal; and
- determine a value representative of the mechanical property, called resting value, based on the selected measurements.

14. An elastography device comprising an electronic unit configured to:
- acquire, at a repetition rate of at least 4 measurements per second and for a duration of at least 3 seconds, measurements of a mechanical property of a region of a body of a subject, said region being a part of a liver or a part of a spleen of the subject;
- obtain a signal representative of the variations of the mechanical property with time, said signal comprising at least part of the measurements of the mechanical property determined;
- in each portion of the signal among at least one portion of the signal, select measurements, among the measurements comprised in the portion of the signal, greater than a threshold associated with the portion of the signal; and
- determine a value representative of a variation of the mechanical property with time, called dynamic value, from the selected measurements.
